## Europäisches Patentamt
## European Patent Office
## Office européen des brevets

(19)

(11) Veröffentlichungsnummer : **0 023 607**
**B1**

(12) # EUROPÄISCHE PATENTSCHRIFT

(45) Veröffentlichungstag der Patentschrift :
22.06.83

(21) Anmeldenummer : 80103996.7

(22) Anmeldetag : 11.07.80

(51) Int. Cl.³ : **C 07 G 7/00, A 61 K 35/16, A 61 K 37/12, A 61 K 7/00, G 01 N 33/86**

(54) Verfahren zur Herstellung einer Kollagenlösung, eine solche Lösung sowie ihre Verwendung.

(30) Priorität : 19.07.79 DE 2929144

(43) Veröffentlichungstag der Anmeldung :
11.02.81 Patentblatt 81/06

(45) Bekanntmachung des Hinweises auf die Patenterteilung : 22.06.83 Patentblatt 83/25

(84) Benannte Vertragsstaaten :
AT CH DE FR IT LI NL

(56) Entgegenhaltungen :
FR A 1 567 174
FR A 2 422 407
US A 3 314 861
US A 3 949 073
CHEMICAL ABSTRACTS, Band 90, Nr. 13, 26. März 1979, Seite 353, Zusammenfassung Nr. 101052g, Columbus, Ohio, US, Y. J. LEGRAND et al. : « Adsorption of factor VIII antigen-activity complex by collagen »
CHEMICAL ABSTRACTS, Band 88, Nr. 3, 16. Januar 1978, Seite 420, Zusammenfassung Nr. 20250b, Columbus, Ohio, US, NYMAN DAG : « Interaction of collagen with the factor VIII anti-gen-activity-von Willebrand factor complex »
CHEMICAL ABSTRACTS, Band 84, Nr. 5, 2. Februar 1976, Seite 137, Zusammenfassung Nr. 26977h, Columbus, Ohio, US, A. SORIA et al. : « Fibrin stabilizing factor (F XIII) and collagen polymerization »

(73) Patentinhaber : BEHRINGWERKE AKTIENGESELL-SCHAFT
Postfach 1140
D-3550 Marburg/Lahn (DE)

(72) Erfinder : Becker, Udo, Dr.
Schmaedelstrasse 17
D-8000 München 60 (DE)
Erfinder : Braun, Konrad
Im Steu 8
D-3557 Ebsdorfergrund (DE)
Erfinder : Heimburger, Norbert, Dr.
Sonnenhang 10
D-3550 Marburg/Lahn (DE)

(74) Vertreter : Meyer-Dulheuer, Karl-Hermann, Dr. et al
HOECHST Aktiengesellschaft Zentrale Patentabteilung Postfach 80 03 20
D-6230 Frankfurt/Main 80 (DE)

### Verfahren zur Herstellung einer Kollagenlösung, eine solche Lösung sowie ihre Verwendung

Die Erfindung betrifft ein Verfahren zur Herstellung einer Kollagenlösung aus menschlichem oder tierischem Gewebe, vor allem aus menschlicher Nabelschnur und Plazenta, und deren Verwendung zur Adsorption der Gerinnungsfaktoren VIII und XIII.

Verfahren zur Herstellung von Kollagen sind bekannt ; beispielsweise aus dem US-Patent 3 949 073, worin eine Kollagenlösung erhalten wird, die beim Erwärmen Fibrillen ausscheidet, die ihrerseits für kosmetische Zwecke oder zur Wundabdeckung verwendet werden. Es ist auch bekannt, daß eine bestimmte Kollagen-Präparation das Faktor VIII-Antigen zu binden vermag, während die Faktor VIII-Aktivität im Überstand bleibt (Nyman, D., Thrombos. Research 11, (1977), 433).

Die bisher beschriebenen Präparate sind für die Gewinnung der genannten Faktoren über ihre selektive und quantitative Adsorption, beispielsweise aus Blutplasma, nicht brauchbar. An einem derartigen Kollagen besteht jedoch wegen der Bedeutung und dem Bedarf an diesen Faktoren für die Behandlung von Gerinnungs- und Wundheilungsstörungen Interesse, insbesondere auch zur Verwendung als Grundmaterial für die Herstellung von Vliesen.

Es wurde nun überraschenderweise gefunden, daß ein unter bestimmten Bedingungen hergestelltes Kollagen die Gerinnungsfaktoren VIII und XIII zu adsorbieren vermag.

Gegenstand der Erfindung ist ein Verfahren zur Herstellung von Kollagen, des dadurch gekennzeichnet ist, daß pepsinbehandeltes Kollagen gegen eine phosphatgepufferte, isotonische Salzlösung vom pH 7-8,5, vorzugsweise vom pH 7,2, die eine basische Aminosäure, vorzugsweise Arginin, in einer Konzentration von 5-20, vorzugsweise 10 mmol/l enthält, dialysiert wird.

Ein geeignetes pepsinbehandeltes Kollagen kann beispielsweise dadurch erhalten werden, daß man menschliches oder tierisches Gewebe, vorzugsweise menschliche Nabelschnur oder Plazenten, mit einer geeigneten Pufferlösung von pH 7-9, die Neutralsalze enthalten kann, vorzugsweise mit einem Tris-Salzsäurepuffer von pH 7,4, der Natriumchlorid und gegebenenfalls einen Komplexbildner, beispielsweise EDTA, Citrat oder Phosphat, vorzugsweise EDTA und/oder einen Proteinaseninhibitor, beispielsweise Soja-Trypsin- oder Kunitzinhibitor oder vorzugsweise Aprotinin, enthält, extrahiert, den abgetrennten Niederschlag mit der gleichen Pufferlösung extrahiert, die jedoch keinen Komplexbildner und keinen Inhibitor enthält, den abgetrennten Niederschlag mit einer wässrigen Lösung einer Säure mit einem pK von 3-5, vorzugsweise mit 0,5 molarer Essigsäure, behandelt, den abgetrennten Niederschlag in einer wässrigen Lösung einer Säure mit einem pK von 3-5, vorzugsweise mit 0,1 molarer Essigsäure, aufnimmt, vorzugsweise mit Salzsäure auf pH 2 bringt, mit Pepsin, vorzugsweise einen Tag bei 4-25 °C, vorzugsweise 25 °C, behandelt, den Überstand abtrennt, die Pepsinbehandlung am Rückstand wiederholt, den Überstand abtrennt, die beiden Überstände vereinigt, durch Erhöhen der Ionenstärke durch Zugabe von Neutralsalz auf 1-1,3 mol/l das Kollagen ausfällt, den abgetrennten Niederschlag in einer Pufferlösung von pH 7-8,5, vorzugsweise einem Tris-Salzsäurepuffer von pH 7,4, der Neutralsalz enthält, löst, gegen den gleichen Puffer dialysiert, gegebenenfalls die Lösung mit einem silikathaltigen Adsorptionsmittel, wie Kaolin, Aerosil, beispielsweise mit Decalite speedplus (Degussa, Frankfurt, Bundesrepublik Deutschland) versetzt und die Kollagen-Lösung abtrennt.

Eine auf diese Weise erhaltene Kollagenlösung scheidet Kollagenfibrillen aus, wenn sie auf etwa 35 bis 40 °C erwärmt wird. Wird die Lösung in Gegenwart der Gerinnungsfaktoren VIII und XIII erwärmt, adsorbieren die ausfallenden Fibrillen diese Faktoren in aktiver Form. Die Temperatur wird so gewählt, daß man eine hohe Ausbeute an aktiven Faktoren erhält. Diese Eigenschaft kann zur weiteren Kennzeichnung des Kollagens dienen, das nach dem erfindungsgemäßen Verfahren erhalten werden kann, dessen einzelne Verfahrensmaßnahmen zum Teil bekannt sind.

Die Erfindung ist für die Herstellung von Konzentraten von Faktor VIII und XIII von Bedeutung, die zur Therapie bestimmter Gerinnungs- und Wundheilungsstörungen eingesetzt werden. Die Therapie der Hämophilie A ist mit den heute verfügbaren Faktor VIII-Konzentraten Kostspiehs, da bei ihrer Herstellung nur etwa 10 % des im Plasma vorhandenen Gehalts an Faktor VIII erhalten werden. Ein Verfahren mit besserer Ausbeute ist daher von Interesse.

Das erfindungsgemäß hergestellte Kollagenpräparat eignet sich weiterhin für die Herstellung von Wundabdeckungen, die zur Blutstillung, speziell auch bei Patienten mit besonderer Blutungsneigung (Hämophilie), beispielsweise nach Zahnextraktionen, geeignet sind. Hierfür wird menschliches Plasma an das Kollagenpräparat adsorbiert, nicht gebundene Plasmateile ausgewaschen und lyophilisiert. Das so gewonnene vliesartige Kollagen enthält alle für die Blutstillung, Fibrinstabilisierung und Wundheilung notwendigen Bestandteile.

Außerdem kann das Kollagenpräparat, an das die Gerinnungsfaktoren adsorbiert sind, verwendet werden als *in vitro* Diagnostikum, um Störungen der Thrombozytenfunktion zu erkennen. Mischt man beispielsweise Blut oder einen Thrombozyten-reichen Plasmaüberstand gesunder Personen mit einer erfindungsgemäß hergestellten Kollagenlösung bei einer Temperatur, bei der Fibrillen entstehen können, verklumpen die Thrombozyten. Bei bestimmten pathologischen Zuständen bleibt die Reaktion aus. Die spontane Fibrillenbildung macht die Kollagenlösung besonders gut als *in vitro* Diagnostikum geeignet. Die bisher verwendeten Suspensionen sind bezüglich der Fibrillengröße nicht standardisierbar.

Eine solche Kollagenlösung oder Kollagensuspension ist auch zur Verwendung für kosmetische Zwecke geeignet, besonders, da es sich um homologes Kollagen handelt.

Testmethoden

Bestimmung des Gerinnungsfaktors VIII :

F VIII : C : Mit der Faktor VIII-Einphasen-Bestimmung mit dem Testkit der Behringwerke AG, Marburg, Bundesrepublik Deutschland

F VIII R : AG : Immunologisch nach LAURELL, C.-B., Anal. Biochem. *10*, 358 (1965)

F VIII R : WF : Funktionell mit Ristocetin nach H.-J. WEISS et al., J. Clin. Invest. *52*, 2708 (1973)

F XIII : Mit der Faktor XIII-Einphasen-Bestimmung mit dem Schnelltest d. Behringwerke AG, Marburg, Bundesrepublik Deutschland

Blutplättchenaggregation : Nach BORN, G.W.R., J. Physiol. (London) 162, 67 (1962)

Immunfluoreszenz : Nach WICK, G., BAUDNER, F. und HERZOG, F. : Immunfluoreszenz, Med. Verlagsgesellschaft Marburg 1976

Hydroxyprolinbestimmung : Nach WOESSNER, J.F., Arch. Biochem. Biophys. *95*, 440 (1961)

Die Erfindung soll durch die nachstehenden Beispiele näher erläutert werden.

Beispiel 1

Herstellung des Kollagens

10 tiefgefrorene Nabelschnüre werden aufgetaut und in 1 cm lange Stücke geschnitten. Die Stücke werden zusammen mit 0,5 l Extraktionspuffer I (0,05 m Tris/HCl, pH 7,4, enthaltend 0,5 m NaCl, 0,01 m EDTA, 250 E/l Antagosan$^{(R)}$) mit einem Messerhomogenisator fein verteilt. Das Gemisch wird zentrifugiert und der Überstand verworfen. Der Niederschlag wird in 2 l Extraktionspuffer I 24 Stunden bei 4 °C gerührt. Es wird zentrifugiert und der Überstand verworfen. Der Niederschlag wird in 0,5 l Extraktions-puffer II, welcher dem Extraktionspuffer I entspricht, jedoch kein Antagosan$^{(R)}$ enthält, suspendiert und nach 30 min erneut zentrifugiert. Nun wird der Niederschlag in 0,5 l 0,5 molarer Essigsäure suspendiert und nach 30 min zentrifugiert. Der Niederschlag wird in 2 l frischer 0,5 molarer Essigsäure aufgenommen und 24 Stunden bei 4 °C gerührt. Nach Zentrifugation und Verwerfen des Überstandes wird der Niederschlag in 3 l 0,1 molarer Essigsäure aufgenommen und unter Rühren mit 1 n Salzsäure unter pH-Kontrolle auf einen pH-Wert von 2 gebracht. Es wird 0,5 g Pepsin (Fa. Serva, 30 Anson-Einheiten pro mg) zugegeben und 24 Stunden bei einer Temperatur von 25 °C gerührt. Es wird zentrifugiert, der Überstand wird aufbewahrt und der Rückstand, mit frischem Pepsin versetzt, unter denselben Bedingungen erneut abgebaut. Unter den genannten Bedingungen erfolgt eine völlige Auflösung des Nabelschnurgewebes. Beide Extrakte werden vereinigt und mit festem NaCl auf eine Konzentration von 0,9 mol/l gebracht. Nach 2-stündigem Rühren wird zentrifugiert und der Überstand verworfen. Das Präzipitat wird in 3 l 0,05 m Tris/HCl, pH 7,4, enthaltend 1 mol/l NaCl gelöst. Nach Auflösung wird gegen denselben Puffer, je zweimal 10 l, für 48 Stunden dialysiert. Die Lösung wird mit 20 g/l Decalite speedplus vermischt und zentrifugiert. Die klare Lösung hat einen Kollagengehalt von 1,8 mg/ml.

1 l dieser Lösung wird gegen 2 mal 5 l Phosphat-gepuffertes physiologisches Kochsalz pH 7,2 (8 g/l Kochsalz, 1,15 g/l Di-natrium-hydrogenphosphat, 0,2 g/l Kaliumdihydrogenphosphat) enthaltend 0,01 mol/l Arginin 24 Stunden bei Raumtemperatur dialysiert und anschließend mit dem Dialysepuffer eine Konzentration von 1 mg Kollagen pro ml eingestellt.

Beispiel 2

5 mal 2 ml menschliches Blutplasma, enthaltend 10 Volumenteile 3,8 %ige Citratlösung als Antikoagulans, werden im Wasserbad auf 37 °C gebracht und mit steigenden Mengen der nach Beispiel 1 erhaltenen Kollagenlösung versetzt, wobei Volumenausgleich durch Phosphat-gepufferte Kochsalzlösung, pH 7,2, enthaltend 0,01 mol/l Arginin, erfolgt. Die zugesetzten Mengen an Kollagen entsprechen 0,1, 0,2, 0,5 und 1,0 mg/ml Plasma. Es wird 10 min bei 37 °C inkubiert und dann 10 min bei 1 500 g zentrifugiert. Die Plasmaüberstände und die präzipitierten Kollagenfibrillen werden durch Abgießen· voneinander getrennt. In den Plasmaüberständen wird die Faktor VIII-Aktivität bestimmt. Die Aktivitäten der drei biologischen Funktionen des Faktor VIII-Moleküls, Faktor VIII-Gerinnungsaktivität (F VIII : C), Faktor VIII-Antigen (F VIII R : AG) und v. Willebrand-Faktor (F VIII R : WF) werden im gleichen Maße bei der höchsten Kollagenkonzentration nahezu quantitativ aus der Lösung entfernt.

Beispiel 3

Der in Beispiel 2 beschriebene Versuch wird mit menschlichem Blutplasma, enthaltend 4 IE Heparin/ml als Antikoagulans, durchgeführt. Die Ergebnisse für F VIII R : AG und F VIII R : WF entsprechen denen von Beispiel 2. F VIII : C kann in Heparin-haltigem Milieu aus methodischen Gründen nicht bestimmt werden.

Beispiel 4

Das nach Beispiel 1 unter Zusatz von 1 mg Kollagen/ml Plasma hergestellte Präzipitat von

3

Kollagenfibrillen wird dreimal mit je 1 ml physiologischer Kochsalzlösung gewaschen und in einer Konzentration von 5 mg/ml in physiologischer Kochsalzlösung suspendiert. Als Vergleichsprobe dient eine gleichermaßen gewaschene Kollagenfibrillensuspension gleicher Konzentration, die durch 10 minütiges Erwärmen eines entsprechenden Volumens der Kollagenlösung auf 37 °C hergestellt wurde.

In einem Gerinnungsautomaten nach Schnitger & Gross (Firma H. Amelung, 4922 Brake, Bundesrepublik Deutschland) werden vier Röhrchen mit je 0,1 ml eines kongenitalen Faktor VIII-Mangelplasmas vorgelegt und auf 37 °C erwärmt. Röhrchen 1 erhält 0,1 ml eines 1 : 5 in physiologischer Kochsalzlösung vorverdünnten Plasmas mit normalem Faktor VIII-Gehalt. Röhrchen 2 erhält 0,1 ml der mit Plasma beladenen und gewaschenen Kollagenfibrillen. Röhrchen 3 erhält 0,1 ml der nicht beladenen adsorbierten Kollagenfibrillen. Röhrchen 4 erhält 0,1 ml physiologische Kochsalzlösung.

Nun gibt man in jedes Röhrchen 0,1 ml einer Kaolin-Plättchenfaktor 3 — Mischung (Pathromtin[R] der Behringwerke AG, Marburg). Nach Mischung wird sechs min. bei 37 °C inkubiert, anschließend jedes Röhrchen mit 0,1 ml einer 0,025 molaren Calciumchloridlösung versetzt und die Gerinnungszeiten bestimmt. Das Ergebnis zeigt Tabelle 1.

Tabelle 1

| Probe (0,1 ml) | Gerinnungszeit (sec) |
|---|---|
| Normalplasma (1 : 5) | 53 |
| Absorbierte Kollagen-suspension (5 mg/ml) | 66 |
| Nicht absorbierte Kollagensuspension (5 mg/ml) | 124 |
| Puffer | 104 |

Aus den Gerinnungszeiten geht hervor, daß eine mit Normalplasma adsorbierte erfindungsgemäß hergestellte Kollagensuspension biologisch aktiven Gerinnungsfaktor VIII enthält.

Beispiel 5

1 ml Kollagenlösung wird analog Beispiel 2 mit 1 ml Citrat-haltigem menschlichem Plasma beladen, die Kollagenfibrillen abzentrifugiert und dreimal mit Phosphatgepufferter physiologischer Kochsalzlösung gewaschen. Die Kollagenfibrillen werden mit für bestimmte Plasmaproteine spezifischen Antiseren vom Kaninchen im indirekten Fluoreszenztest auf adsorbierte Plasmaproteine untersucht. Mit Kaninchen-Normalserum wird eine negative, mit Anti-Humanfibrinogen, Anti-Human-Immunglobulin und Anti-Humanalbumin eine sehr schwache Reaktion gefunden. Mit Anti-Human-Faktor VIII-Serum wird eine sehr starke Reaktion gefunden.

Dieses Beispiel zeigt die selektive und spezifische Bindung von Faktor VIII, der zum Beispiel gegenüber Albumin in 4 400-fach niedrigerer Konzentration im Plasma enthalten ist.

Beispiel 6

Beispiel 5 wird mit dem Plasma eines Patienten mit v. Willebrand'scher Krankheit ausgeführt, dessen Faktor VIII-Gehalt weniger als 10 % der Norm beträgt. Mit Antiserum gegen Faktor VIII wird nur eine schwache Reaktion beobachtet.

Beispiel 7

Nach Beispiel 2 wird eine Adsorption von menschlichem Citratplasma mit steigenden Mengen von Kollagen durchgeführt. Die Plasmaüberstände werden auf ihren Gehalt an Faktor XIII untersucht. Der verwendete Test ist ein qualitatives Verfahren, das die Aktivitätsbereiche in % bezogen auf Normalplasma angibt. Unter Berücksichtigung der Ausgangsverdünnung von 1 : 2 werden die in Tabelle 2 angegebenen %-Werte gefunden.

Tabelle 2

| Kollagenmenge (mg/ml) | Faktor XIII-Bereich in % der Norm |
|---|---|
| 0,0 | 100 - 150 |
| 0,1 | 75 - 100 |
| 0,2 | 50 - 75 |
| 0,5 | 25 - 50 |
| 1,0 | < 25 |

Beispiel 8

In einem Aggregometer nach Born mit Schreiber (Fa. Braun, Melsungen, Bundesrepublik Deutschland) werden 1 ml blutplättchenreiches Citratplasma in die Küvette gefüllt. Photometer und Schreiber werden auf 0 % Transmission eingestellt. Es wird magnetisch gerührt und Temperaturangleich auf 37 °C abgewartet. Dann wird 1 µl der klaren Kollagenlösung nach Beispiel 1, entsprechend 1 µg Kollagen, mit einer Mikroliterspritze zugegeben. Innerhalb von zwei Minuten erfolgt eine Zusammenballung (Aggregation) der Blutplättchen zu großen Aggregaten, was zu einer starken Transmissionszunahme führt, welche von dem Schreiber aufgezeichnet wird. Diese Reaktion ist für die *in vitro* Diagnose von Gerinnungsstörungen geeignet.

Beispiel 9

Die Kollagenlösung wird auf etwa 35 bis 40 °C erwärmt, so daß sie Fibrillen ausscheidet. Die gelartige Masse wird lyophilisiert, wodurch ein stabiles weißes Kollagenvlies erhalten wird. Der Kollagenlösung können Faktor VIII, Faktor XIII und/oder Fibrinogen zugesetzt worden sein.

**Ansprüche**

1. Verfahren zur Herstellung einer Kollagenlösung, dadurch gekennzeichnet, daß pepsinbehandeltes Kollagen gegen eine phosphat-gepufferte, isotonische Salzlösung vom $p_H$ 7-8,5 dialysiert wird, die eine basische Aminosäure in einer Konzentration von 5 bis 20 mmol/l enthält.

2. Eine Kollagenlösung erhältlich nach Anspruch 1, dadurch gekennzeichnet, daß sie beim Erwärmen auf eine Temperatur von etwa 35 bis 40° Kollagenfibrillen ausscheidet.

3. Verwendung einer Kollagenlösung nach Anspruch 2 zur Adsorption der Gerinnungsfaktoren VIII und XIII aus diese enthaltenden Flüssigkeiten.

4. Verwendung einer Kollagenflüssigkeit nach Anspruch 2 zur Herstellung von Kollagenfibrillen, an welche die Gerinnungsfaktoren VIII und XIII adsorbiert sind, und die gegebenenfalls Fibrinogen enthalten.

5. Verwendung einer Kollagenlösung nach Anspruch 2 als *in vitro* Diagnostikum.

**Claims**

1. Process for the manufacture of a collagen solution, which comprises dialyzing a pepsin-treated collagen against a phosphate-buffered isotonic salt solution of pH 7-8.5 containing a basic amino acid in a concentration from 5 to 20 mmols/l.

2. A collagen solution as obtained according to claim 1, characterized in that collagen fibrillae precipitate therefrom upon heating to a temperature of approximately 35 to 40 °C.

3. Use of a collagen solution according to claim 2 for adsorbing the coagulation factor VIII and XIII from liquids wherein the latter are contained.

4. Use of a collagen liquid according to claim 2 for the manufacture of collagen fibrillae, whereto the coagulation factors VIII and XIII have been adsorbed and which may contain fibrinogen.

5. Use of a collagen solution according to claim 2 as diagnostic agent *in vitro*.

**Revendications**

1. Procédé de préparation d'une solution de collagène, caractérisé en ce que l'on dialyse du collagène traité par la pepsine contre une solution saline isotonique tamponnée au phosphate, de pH 7-8,5, qui contient un amido-acide basique en une concentration de 5 à 20 mmol/l.

2. Solution de collagène obtenue selon la revendication 1, caractérisée en ce que des fibrilles de collagène se séparent de cette solution par chauffage à une température d'environ 35 à 40 °C.

3. Utilisation d'une solution de collagène selon la revendication 2, pour l'adsorption des facteurs de coagulation VIII et XIII de liquides les contenant.

4. Utilisation d'un liquide à base de collagène selon la revendication 2, pour la préparation de fibrilles de collagène sur lesquelles sont adsorbés les facteurs de coagulation VIII et XIII, et contenant éventuellement du fibrinogène.

5. Utilisation d'une solution de collagène selon la revendication 2, comme agent de diagnostic *in vitro*.